# NEW EUROPEAN PATENT SPECIFICATION

(11) **EP 2 021 055 B2**
(45) Date of publication and mention of the opposition decision: **21.02.2018**
(45) Mention of the grant of the patent: 02.03.2011
(21) Application number: 07733018.1
(22) Date of filing: 30.05.2007
(51) Int. Cl.: A61M 5/20

(54) **INJECTION DEVICE**
INJEKTIONSVORRICHTUNG
DISPOSITIF D'INJECTION

(30) Priority: 01.06.2006 GB 0610859
(43) Date of publication of application: 11.02.2009
(73) Proprietor: Cilag GmbH International, 6300 Zug (CH)
(72) Inventor: BARROW-WILLIAMS, Timothy Donald, St Albans, Hertfordshire AL1 1PL (GB)
(74) Representative: Tunstall, Christopher Stephen
(86) International application number: PCT/GB2007/002002
(87) International publication number: WO 2007/138319

(56) References cited:
- WO-A-2005/025636
- WO-A-2006/063124
- WO-A1-94/21316
- CH-A- 518 102
- US-A- 4 231 368
- US-A1- 2003 233 070
- US-B1- 6 387 078

## Description

### FIELD OF THE INVENTION

The present invention relates to an injection device of the type that receives a syringe, extends it, discharges its contents and then retracts it automatically.

### BACKGROUND OF THE INVENTION

Previously known injection devices are shown in WO95135126 and EP0516473 and tend to employ a drive spring and some form of release mechanism that releases the syringe from the influence of the drive spring once its contents are supposed to have been discharged to allow it to be retracted by a return spring.

The high impact forces associated with the spring-operated mechanisms of such devices call lead to mechanical failure of various components. This causes improper operation of the device and the user may not receive the correct dose of the drug to be administered. The syringe itself is often manufactured from glass and is therefore brittle and liable to fracture. The problem of syringe breakage during operation of the device is discussed in a co-pending UK patent application, published as GB 2414401.

Such devices also incorporate a delay mechanism as part of the multi-component drive system that advances the syringe from the housing of the device and pushes its needle into a user's body by application of force to the rear of the syringe stopper. This may fail during a firing cycle by brittle fracture caused by transmission of an impact force due to sudden deceleration of the syringe carrier relative to the case nose as the two components contact.

United States Patent Nos. 4231368 and 6387078 each disclose an injection device having a damping element which acts as a buffer between the syringe and device housing when the syringe contacts the housing during operation.

International patent application publication no. WO2005/025636 discloses an injection device with a damping element which acts between components of the drive mechanism to reduce the force of the impact acting on the syringe during operation of the injection device.

### SUMMARY OF THE INVENTION

The injection device of the present invention is designed to overcome this and other problems.

In view of the foregoing and in accordance with a first aspect of the invention, there is provided an injection device as defined by claim 1.

The damping element acts as a cushion to reduce the transmission of an impact force to the components of the drive, due to sudden deceleration of the syringe carrier relative to the restraining component as the two components come into contact when the syringe reaches its extended position. The peak loading in these components is thereby reduced and their fracture can be prevented. The damping element also reduces the noise, which may be distressing to a user of the device, produced when the syringe carrier and restraining component come into contact and reduces the pain suffered by a user upon operation of the device.

In an embodiment of the present invention, the position of the restraining component may be fixed relative to the housing. Alternatively, the restraining component is integrally formed with the housing.

The syringe carrier provides an interface between the syringe and the restraining component and, preferably, the syringe acts upon the syringe carrier to advance it. Advantageously, the interaction of the syringe carrier and the restraining component restrains the advancement of the syringe beyond its extended position.

The syringe carrier may comprise a cylindrical section having an external diameter and the restraining component may comprise a cylindrical section having an internal diameter, wherein the external diameter of the cylindrical section of the syringe carrier is less than the internal diameter of the cylindrical section of the restraining component. Preferably, the syringe carrier further comprises a flange with an external diameter that is larger than the internal diameter of the restraining component. The restraining component may act upon the flange of the syringe carrier to restrain its advancement as the syringe reaches its extended position.

The damping element may be positioned between the restraining component and the flange of the syringe carrier. Alternatively, the damping element may be located at the end of the syringe carrier through which the discharge nozzle of the syringe passes.

The damping element may be integrally formed with either the syringe carrier or the restraining component. Preferably, the damping element integrally formed with the syringe carrier. This may be achieved by moulding the damping element into the syringe carrier.

The damping element may be annular in shape and is preferably a thermoplastic elastomer that may be selected from Santoprene®, Evoprene® or polyurethane. Most preferably, the damping element is made of Santoprene®.

Preferably, the means for biasing the syringe acts between the restraining component and the flange of the syringe carrier. The restraining component may have a region of reduced internal diameter that is acted upon by the biasing means.

Preferably, the restraining component is a sleeve that substantially surrounds the syringe carrier.

### BRIEF DESCRIPTION OF THE DRAWINGS

The invention will now be described by way of example with reference to the accompanying drawings, in which:
Figure 1 shows a perspective view of an injection device according to the present invention;
Figure 2 shows a side view of the injection device of figure 1 with the housing of the injection device removed,
Figure 3 shows a side view of the injection device of figure 1 with further components removed;
Figure 4 shows a side view of the sleave, the return spring, the syringe carrier and the damping element of the injection device of figure 1; and
Figure 5 shows a side view of the sleeve, the return spring, the syringe carrier and the damping element of an alternative injection device of the present invention.

### DETAILED DESCRIPTION OF THE DRAWINGS

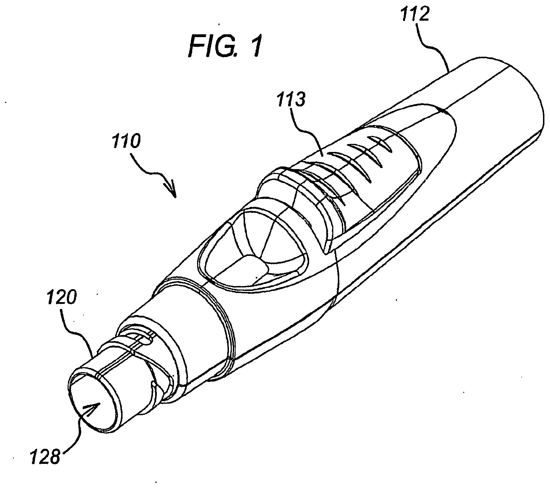

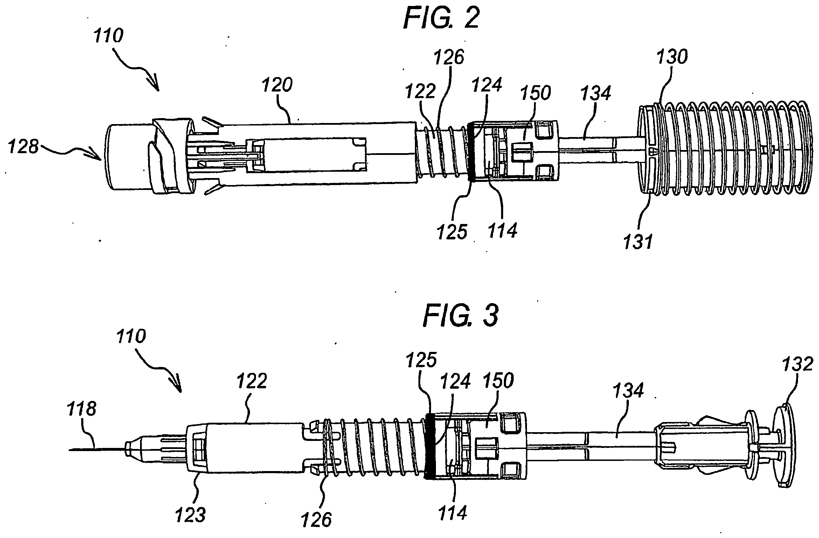

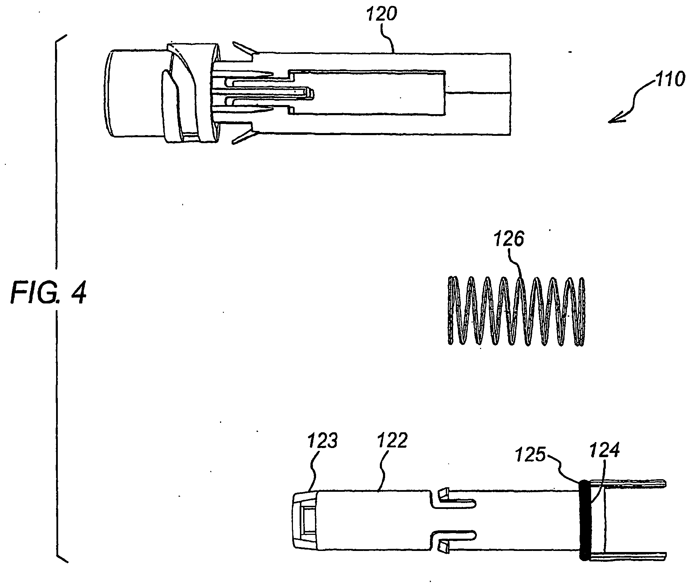

Figures 1 to 4 show an injection device 110 according to a first embodiment of the present invention. The injection device 110 has a syringe 114 contained within a housing 112. The syringe 114 comprises a needle 118 and is housed within a syringe carrier 122, which in turn sits partially inside a sleeve 120.

The syringe carrier 122 has a first end 123 that supports the discharge end of the syringe 114. At the other end of the syringe carrier 122 is a flange 124 against which a return spring 126 is biased. The return spring 126 acts between the flange 124 and a region of reduced internal diameter (not shown) of the sleeve 120 to bias the syringe 114 from an extended position, in which the needle 118 expends from the aperture 128, to a retracted position, in which the needle 118 is contained within the housing 112. A damping element 125 is integrally formed with the syringe carrier 122 in front of flange 124. The damping element 125 is annular in shape and is fabricated from Santoprene®, a thermoplastic elastomer.

The drive takes the form of a compression drive spring 130. Drive from the derive spring 130 is transmitted via a multi-component drive to the piston of the syringe 114 to advance the syringe 114 from its retracted position to its extended position and discharge its contents through the needle 118. The drive accomplishes this task by acting directly upon the syringe 114 and its contents. Static friction between the drive element 134 and the syringe body initially ensures that they advance together, until the return spring 126 bottoms out.

The multi-component drive between the drive spring 130 and the syringe 114 consists of three principal components. A drive sleeve 131 takes drive from the drive spring 130 and transmits it to a first drive element 132, This in turn transmits drive to the second drive element 134 already mentioned.

The drive element 132 includes a hollow stem (not shown), the inner cavity of which forms a collection chamber in communication with a vent that extends from the collection chamber through the end of the stem. The second drive element 134 includes a blind bore (not shown) that is open at one end to receive the stem and closed at the other. The bore and the stem define a fluid reservoir, within which a damping fluid is contained.

A trigger 113 is provided on one side of the housing 112. The trigger 113, when operated, serves to decouple the drive sleeve 131 from the housing 112, allowing it to move relative to the housing 112 under the influence of the drive spring 130. The operation of the device 110 is then as follows.

Initially, the drive spring 130 moves the drive sleeve 131, the drive sleeve 131 moves the first drive element 132 and the first drive element 132 moves the second drive element 134. The second drive element 134 moves and, by virtue of static friction and hydrostatic forces acting through the contents of the syringe 114, moves the syringe body against the action of the return spring 126. The syringe body moves the syringe carrier 122, which compresses the return spring 126 via the flange 124. The needle 118 emerges from the exit aperture 128 of the housing 112. This continues until the return spring 126 bottoms out or the syringe body meets some other obstruction (not shown) that retards its motion.

At the point when the return spring 126 bottoms out, the damping element 125 acts between the sleeve 120, via its region of reduced internal diameter, and the syringe carrier 122, via its flange 124, to absorb some of the energy of the impact. The damping clement 125 has the effect of reducing the transmission of an impact force, caused by the sudden deceleration of the syringe carrier 122 relative to the sleeve 120 as the two components contact, to the drive mechanism, specifically to the first drive element 132. This feature improves the reliability of the device 110 by reducing the peak loading in the first drive element 132 and preventing its fracture. The damping element 125 givers the additional advantage of reducing any noise, which may be disconcerting for a user, that is produced during operation of the device 1110 as the flange 124 of the syringe currier 122 strikes the sleeve 120. The damping element 125 also serves to reduce the pain suffered by a user upon operation of the device 110.

The static friction between the second drive element 134 and the syringe body and the hydrostatic forces acting through the contents of the syringe 114 are not sufficient to resist the full drive force developed by the drive spring 130, so at this point the second drive element 134 begins to move within the syringe body and its contents begin to he discharged. Dynamic friction between the second drive element 134 and the syringe body and hydrostatic and hydrodynamic forces now acting through the contents of the syringe 114 are, however, sufficient to retain the return spring 126 in its compressed state, so the needle 118 remains extended.

Before the second drive element 134 reaches the end of its travel within the syringe body, so before the contents of the syringe 114 have fully discharged, flexible latch arms linking the first and second drive elements 132, 134 reach a constriction within the housing 112 formed by an annular portion 150 at the end of the syringe carrier 122 that includes the flange 124. The constriction moves the flexible latch arms to a position so that they no longer couple the first drive element 132 to the second drive element 134. Once this happens, the first drive element 132 no longer acts on the second drive element 134, allowing the first drive element 132 to move relative to the second drive element 134.

Because the damping fluid is contained within a reservoir defined between the end of the first drive clement 132 and the blind bore in the second drive element 134, the volume of the reservoir will trend to decrease as the first drive element 132 moves relative to the second drive element 134 when the former is acted upon by the drive spring 130. As the reservoir collapsus, damping fluid is forced through the vent into the collection chamber. Thus, once the flexible latch arms have been released, some of the force exerted by the drive spring 130 does work on the damping fluid, causing it to flow though the constriction formed by the vent; the remainder acts hydrostatically through the fluid and through friction between the first and second drive elements 132, 134, thence via the second drive element 134. Consequently, the second drive element 134 continues to move within the syringe body and the contents of the syringe 114 continue to be discharged. Losses associated with the flow of the damping fluid do not attenuate the force acting on the syringe body to a great extent. Thus, the return spring 126 remains compressed and the needle 118 remains extended.

After a time, the second drive element 134 completes its travel within the syringe body and can go no further. At this point, the contents of the syringe 114, are completely discharged and the force exerted by the drive spring 130 acts to retain the second drive element 134 in its terminal position and to continue to cause the damping fluid to flow though the vent, allowing the first drive element 132 to continue its movement.

Before the reservoir of fluid is exhausted, flexible latch arms linking the drive sleeve 131 with the first drive element 132 reach another constriction within the housing 112. The constriction moves the flexible latch arms so that they no longer couple the drive sleeve 131 to the first drive element 132. Once this happens, the drive sleeve 131 no longer acts on the first drive element 132, allowing them to move relative to each other. At this point, the forces developed by the drive spring 130 are no longer being transmitted to the syringe 114. The only force acting on the syringe 114 will be the return force from the return spring 126 which acts on the end 123 of the syringe 114 nearest to the needle 118 via the flange 124 and the syringe carrier 122. Consequently, the syringe 114 is returned to its retracted position and the injection cycle is complete.

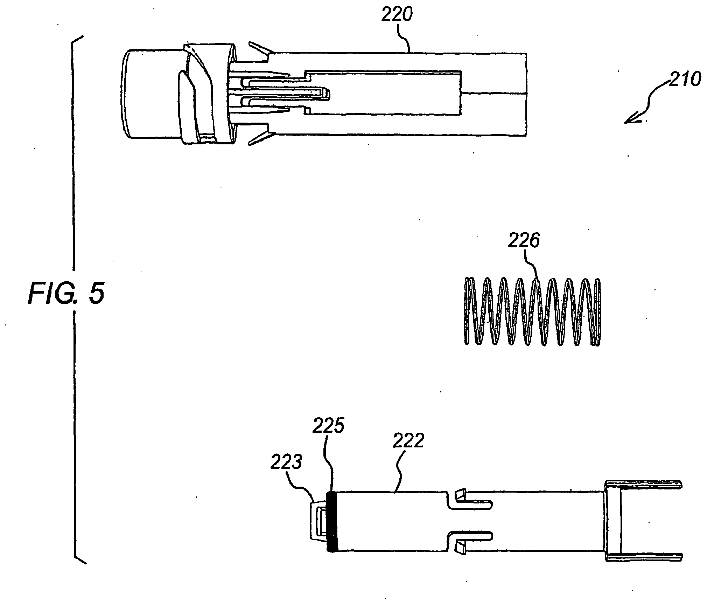

Figure 5 shows components of an injection device 210 according to a second embodiment of the present invention. The device 210 includes a sleeve 220 in which is substantially positioned a syringe carrier 222 having a damping element 225 co-moulded with a first end 223 of the syringe carries which is located nearest to an exit aperture of the device 210. Contact between an interface surface on the sleeve 220 and the first end 223 of the syringe carrier 222 restrains the syringe as it reaches its extended position. The damping element 225 acts between the sleeve 220 and the syringe carrier 222 at this point to reduce transmission of an impact force to a first drive element in a similar fashion to that previously described.

It will of course be understood that the present invention has been described above purely by way of example and modifications of detail can be made within the scope of the invention.

## Claims

1. An injection device (110) comprising:
a housing (112) adapted to receive a syringe (114) having a discharge nozzle, so that the syringe (114) is movable between a retracted position in which the discharge nozzle is contained within the housing (112) and an extended position in which the discharge nozzle extends from the housing (112) through an exit aperture;
a drive that acts upon the syringe (114) to advance it from its retracted position to its extended position and discharge its contents through the discharge nozzle,
a syringe carrier (122) that advances with the syringe (114);
a restraining component that restrains the advancement of the syringe carrier (122) as the syringe (114) reaches its extended position;
means for biasing the syringe from its extended position to its retracted position, wherein the means for biasing the syringe is a return spring; and
a damping element (125) that acts between the syringe carrier (122) and the restraining component at the point at which the return spring bottoms out to thereby absorb impact energy.

2. The injection device (110) of claim 1 wherein the position of the restraining component is fixed relative to the housing (112).

3. The injection device (110) of claim 2 wherein the restraining component is integrally formed with the housing (112).

4. The injection device (110) of any preceding claim wherein the syringe carrier (122) provides an interface between the syringe (114) and the restraining component.

5. The injection device (110) of any preceding claim wherein the syringe (114) acts upon the syringe carrier (122) to advance it.

6. The injection device (110) of any preceding claim wherein the interaction of the syringe carrier (122) and the restraining components restrains the advancement of the syringe (114) beyond its extended position.

7. The injection device (110) of any preceding claim wherein the syringe carrier (122) comprises a cylindrical section having an external diameter and the restraining component comprises a cylindrical section having an internal diameter, wherein the external diameter of the cylindrical section of the syringe carrier (122) is less than the internal diameter of the cylindrical section of the restraining component.

8. The injection device (110) of any preceding claim wherein the syringe carrier (122) further comprises a flange with an external diameter that is larger than the internal diameter of the restraining component.

9. The injection device (110) of claim 8 wherein the restraining component interacts with the flange of the syringe carrier (122) to restrain its advancement as the syringe (114) reaches its extended position.

10. The injection device (110) of claim 9 wherein the damping element (125) is positioned between the restraining component and the flange of the syringe carrier (122).

11. The injection device (110) of any of claims 1-8 wherein the syringe carrier (122) has a first end through which the discharge nuzzle of the syringe (114) passes, wherein the damping element (125) is located at the first end.

12. The injection device (110) of any preceding claim wherein the damping element (125) is integrally formed with either the syringe carrier (122) or the restraining component.

13. The injection device (110) of claim 12 wherein the damping element (125) is integrally formed with the syringe carrier (122).

14. The injection device (110) of any preceding claim wherein the damping element (125) is annular in shape.

15. The injection device (110) of any preceding claim wherein the damping element (125) is a thermoplastic elastomer.

16. The injection device (110) of claim 1 wherein the syringe carrier (122) comprises a support for carrying the means for biasing the syringe (114).

17. the injection device (110) of claim 16 wherein the means for biasing the syringe (114) act between the retraining components and the flange of the syringe carrier (122).

18. The injection device (110) of claim 17 wherein the restraining component has a. region of reduced internal diameter that is acted upon by the biasing means.

19. The injection device (110) of claim 18 wherein the restraining component is a sleeve that substantially surrounds the syringe carrier (122).

## Patentansprüche

1. Injektionsvorrichtung (110), aufweisend:
ein Gehäuse (112), welches ausgestaltet ist, um eine Spritze (114) aufzunehmen, welche eine Auslassdüse aufweist, so dass die Spritze (114) zwischen einer zurückgezogenen Position, in welcher die Auslassdüse innerhalb des Gehäuses (112) aufgenommen ist, und einer ausgefahrenen Position, in welcher die Auslassdüse sich von dem Gehäuse (112) durch eine Auslassöffnung erstreckt, beweglich ist;
einen Antrieb, welcher auf die Spritze (114) einwirkt, um sie von ihrer zurückgezogenen Position in ihre ausgefahrene Position zu bewegen und um ihre Inhalte durch die Auslassdüse auszugeben,
einen Spritzenträger (122), welcher die Spritze (114) vorwärts bewegt;
eine Beschränkungskomponente, welche die Vorwärtsbewegung des Spritzenträgers (122) beschränkt, wenn die Spritze (114) ihre ausgefahrene Position erreicht;
Mittel zum Vorspannen der Spritze von ihrer ausgefahrenen Position zu ihrer zurückgezogenen Position, wobei das Mittel zum Vorspannen der Spritze eine Rückstellfeder ist; und
ein Dämpfungselement (125), welches zwischen dem Spritzenträger (122) und der Beschränkungskomponente an dem Punkt wirkt, an dem die Rückstellfeder zum Absorbieren von Aufprallenergie voll einfedert.

2. Injektionsvorrichtung (110) gemäß Anspruch 1, wobei die Position der Beschränkungskomponente relativ zu dem Gehäuse (112) fest ist.

3. Injektionsvorrichtung (110) gemäß Anspruch 2, wobei die Beschränkungskomponente einstückig mit dem Gehäuse (112) gebildet ist.

4. Injektionsvorrichtung (110) gemäß einem der vorhergehenden Ansprüche, wobei der Spritzenträger (122) eine Schnittstelle zwischen der Spritze (114) und der Beschränkungskomponente bildet.

5. Injektionsvorrichtung (110) gemäß einem der vorhergehenden Ansprüche, wobei die Spritze (114) auf den Spritzenträger (122) einwirkt um ihn vorwärts zu bewegen.

6. Injektionsvorrichtung (110) gemäß einem der vorhergehenden Ansprüche, wobei das Zusammenwirken des Spritzenträgers (122) und der Beschränkungskomponenten die Vorwärtsbewegung der Spritze (114) jenseits ihrer ausgefahrenen Position beschränkt.

7. Injektionsvorrichtung (110) gemäß einem der vorhergehenden Ansprüche, wobei der Spritzenträger (122) einen zylindrischen Abschnitt aufweist, welcher einen äußeren Durchmesser aufweist, und wobei die Beschränkungskomponente einen zylindrischen Abschnitt aufweist, welcher einen inneren Durchmesser aufweist, wobei der äußere Durchmesser des zylindrischen Abschnitts des Spritzenträgers (122) kleiner ist als der innere Durchmesser des zylindrischen Abschnitts der Beschränkungskomponente.

8. Injektionsvorrichtung (110) gemäß einem der vorhergehenden Ansprüche, wobei der Spritzenträger (122) weiterhin einen Flansch mit einem äußeren Durchmesser aufweist, welcher größer ist als der innere Durchmesser der Beschränkungskomponente.

9. Injektionsvorrichtung (110) gemäß Anspruch 8, wobei die Beschränkungskomponente mit dem Flansch des Spritzenträgers (122) zusammenwirkt, um dessen Vorwärtsbewegung zu beschränken, wenn die Spritze (114) ihre ausgefahrene Position erreicht hat.

10. Injektionsvorrichtung (110) gemäß Anspruch 9, wobei das Dämpfungselement (125) zwischen der Beschränkungskomponente und dem Flansch des Spritzenträgers (122) positioniert ist.

11. Injektionsvorrichtung (110) gemäß einem der Ansprüche 1 bis 8, wobei der Spritzenträger (122) ein erstes Ende aufweist, durch welches die Auslassdüse der Spritze (114) hindurchläuft, wobei das Dämpfungselement (125) am ersten Ende angeordnet ist.

12. Injektionsvorrichtung (110) gemäß einem der vorhergehenden Ansprüche, wobei das Dämpfungselement (125) entweder mit dem Spritzenträger (122) oder der Beschränkungskomponente einstückig ausgebildet ist.

13. Injektionsvorrichtung (110) gemäß Anspruch 12, wobei das Dämpfungselement (125) einstückig mit dem Spitzenträger (122) ausgebildet ist.

14. Injektionsvorrichtung (110) gemäß einem der vorhergehenden Ansprüche, wobei das Dämpfungselement (125) eine ringförmige Form aufweist.

15. Injektionsvorrichtung (110) gemäß einem der vorhergehenden Ansprüche, wobei das Dämpfungselement (125) ein thermoplastisches Elastomer ist.

16. Injektionsvorrichtung (110) gemäß Anspruch 1, wobei der Spritzenträger (122) eine Unterstützung zum Tragen des Mittels zum Vorspannen der Spritze (114) aufweist.

17. Injektionsvorrichtung (110) gemäß Anspruch 16, wobei das Mittel zum Vorspannen der Spritze (114) zwischen der Beschränkungskomponente und dem Flansch des Spritzenträgers (122) wirkt.

18. Injektionsvorrichtung (110) gemäß Anspruch 17, wobei die Beschränkungskomponente eine Region mit vermindertem innerem Durchmesser aufweist, auf welche das Vorspannmittel einwirkt.

19. Injektionsvorrichtung (110) gemäß Anspruch 18, wobei die Beschränkungskomponente eine Hülse ist, welche im Wesentlichen den Spritzenträger (122) umgibt.

## Revendications

1. Dispositif d'injection (110) comprenant :
un logement (112) adapté pour recevoir une seringue (114) dotée d'une buse de décharge, de sorte que la seringue (114) soit mobile entre une position rétractée, dans laquelle la buse de décharge est contenue dans le logement (112), et une position étendue dans laquelle la buse de décharge s'étend depuis le logement (112) à travers une ouverture de sortie ;
une commande qui agit sur la seringue (114) pour l'avancer de sa position rétractée à sa position étendue et décharger son contenu à travers la buse de décharge,
un support de seringue (122) qui avance avec la seringue (114) ;
un composant de limitation qui limite la progression du support de seringue (122) quand la seringue (114) atteint sa position étendue ;
un moyen pour solliciter la seringue (114) de sa position étendue à sa position rétractée, le moyen pour solliciter la seringue étant un ressort de rappel ; et
un élément d'amortissement (125) qui agit entre le support de seringue (122) et le composant de limitation à l'endroit auquel le ressort de rappel arrive en bout de course pour ainsi absorber l'énergie d'impact.

2. Dispositif d'injection (110) selon la revendication 1, dans lequel la position du composant de limitation est fixe relativement au logement (112).

3. Dispositif d'injection (110) selon la revendication 2, dans lequel le composant de limitation est intégralement formé avec le logement (112).

4. Dispositif d'injection (110) selon l'une quelconque des revendications précédentes, dans lequel le support de seringue (122) fournit une interface entre la seringue (114) et le composant de limitation.

5. Dispositif d'injection (110) selon l'une quelconque des revendications précédentes, dans lequel la seringue (114) agit sur le support de seringue (122) pour l'avancer.

6. Dispositif d'injection (110) selon l'une quelconque des revendications précédentes, dans lequel l'interaction entre le support de seringue (122) et le composant de limitation limite la progression de la seringue (114) au-delà de sa position étendue.

7. Dispositif d'injection (110) selon l'une quelconque des revendications précédentes, dans lequel le support de seringue (122) comprend une section cylindrique ayant un diamètre extérieur et le composant de limitation comprend une section cylindrique ayant un diamètre intérieur, dans lequel le diamètre extérieur de la section cylindrique du support de seringue (122) est inférieur au diamètre intérieur de la section cylindrique du composant de limitation.

8. Dispositif d'injection (110) selon l'une quelconque des revendications précédentes, dans lequel le support de seringue (122) comprend en outre une bride ayant un diamètre extérieur qui est supérieur au diamètre intérieur du composant de limitation.

9. Dispositif d'injection (110) selon la revendication 8, dans lequel le composant de limitation interagit avec la bride du support de seringue (122) pour limiter sa progression quand la seringue (114) atteint sa position étendue.

10. Dispositif d'injection (110) selon la revendication 9, dans lequel l'élément d'amortissement (125) est positionné entre l'élément de limitation et la bride du support de seringue (122).

11. Dispositif d'injection (110) selon l'une quelconque des revendications 1-8, dans lequel le support de seringue (122) a une première extrémité à travers laquelle passe la buse de décharge de la seringue (114), dans lequel l'élément d'amortissement (125) est situé à la première extrémité.

12. Dispositif d'injection (110) selon l'une quelconque des revendications précédentes, dans lequel l'élément d'amortissement (125) est intégralement formé avec le support de seringue (122) ou le composant de limitation.

13. Dispositif d'injection (110) selon la revendication 12, dans lequel l'élément d'amortissement (125) est intégralement formé avec le support de seringue (122).

14. Dispositif d'injection (110) selon l'une quelconque des revendications précédentes, dans lequel l'élément d'amortissement (125) est de forme annulaire.

15. Dispositif d'injection (110) selon l'une quelconque des revendications précédentes, dans lequel l'élément d'amortissement (125) est un élastomère thermoplastique.

16. Dispositif d'injection (110) selon la revendication 1, dans lequel le support de seringue (122) comprend un support pour porter le moyen permettant de solliciter la seringue (114).

17. Dispositif d'injection (110) selon la revendication 16, dans lequel le moyen pour solliciter la seringue (114) agit entre le composant de limitation et la bride du support de seringue (122).

18. Dispositif d'injection (110) selon la revendication 17, dans lequel le composant de limitation a une zone de diamètre intérieur réduite sur laquelle agit le moyen de sollicitation.

19. Dispositif d'injection (110) selon la revendication 18, dans lequel le composant de limitation est un manchon qui entoure sensiblement le support de seringue (122).
